(19) Europäisches Patentamt
European Patent Office
Office européen des brevets

(11) Publication number: 0 243 404 B1

(12) EUROPEAN PATENT SPECIFICATION

(45) Date of publication of patent specification: **18.08.93**

(51) Int. Cl.$^5$: **C12M 1/40**, C12M 1/12

(21) Application number: **86906207.5**

(22) Date of filing: **08.10.86**

(86) International application number:
**PCT/US86/02089**

(87) International publication number:
**WO 87/02381 (23.04.87 87/09)**

# (54) METHOD FOR CONFINING A CATALYST IN MULTIPHASE MEMBRANE REACTOR SYSTEMS.

(30) Priority: **11.10.85 US 786764**
**01.10.86 US 912595**

(43) Date of publication of application:
**04.11.87 Bulletin 87/45**

(45) Publication of the grant of the patent:
**18.08.93 Bulletin 93/33**

(84) Designated Contracting States:
**AT BE CH DE FR GB IT LI LU NL SE**

(56) References cited:
**EP-A- 0 120 285**
**US-A- 4 266 026**
**US-A- 4 307 195**
**US-A- 4 356 267**
**US-A- 4 437 994**
**US-A- 4 598 056**

(73) Proprietor: **SEPRACOR, INC.**
**33 Locke Drive**
**Marlborough, MA 01752(US)**

(72) Inventor: **MATSON, Stephen, L.**
**15 Withington Lane**
**Harvard, MA 01451(US)**

(74) Representative: **Timmermans, Anthonius C.Th., Ir. et al**
**European Patent Attorneys Octrooibureau Zuid P.O. Box 2287**
**NL-5600 CG Eindhoven (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3. 10/3.6/3.3. 1)

## Description

### TECHNICAL FIELD

The present invention relates to a method in which enzymes and other catalysts are confined within membranes for use as membrane reactors in multiphase reaction systems. The invention also relates to a variety of membranes having different solvent-wetting character and configurations, and to methods for charging such membranes with catalysts and for regenerating the membrane reactors once the catalysts therein confined have become inactivated through use.

### BACKGROUND OF THE INVENTION

A method and apparatus are disclosed for the confinement or containment of a catalyst either in an asymmetric membrane or in a composite membrane structure, which is subsequently used to conduct a chemical or biochemical reaction in which multiple phases (e.g. organic and aqueous) are involved.

"Immobilization" on solid-phase supports of otherwise homogeneous catalysts (including, but not limited to enzymes, whole cells, and non-biological catalysts such as various metal-containing coordination compounds) is useful because immobilization simplifies the separation of reaction products from catalyst and it facilitates the recovery and reuse of catalyst, which frequently is too expensive for one-time use. However, as discussed below, such catalyst immobilization is often accomplished by covalently attaching the catalyst to the support, generally via irreversible, covalent linking chemistry. As a result, when a supported catalyst becomes deactivated, as biocatalysts such as enzymes inevitably do, it is difficult if not impossible to replace the catalyst without at the same time replacing the support matrix. Replacement of the catalyst/support combination can be a considerably more expensive proposition than replacement of the catalyst component alone because of the cost of the immobilization chemistry and of the support itself.

Typical supports are membrane structures and particulate media such as microporous and gel-type beads. Membrane supports are attractive because membrane reactors have a number of performance advantages relative to packed-bed reactors employing catalysts bound to particulate support media. However, they have the significant disadvantage that membrane supports are expensive relative to particulate media. Accordingly, the costs associated with periodically replacing membrane-supported catalysts can be significantly higher than is the case with particulate supports.

A significant improvement in membrane bioreactor economics would result from the localization of catalysts in a membrane structure in such a way as to (1) provide effective containment of the catalyst in the membrane, (2) permit high effective catalyst loadings to be realized, and (3) make possible simple catalyst replacement by avoiding the covalent attachment of catalyst to the membrane surface. Such a technology would significantly reduce the cost of catalyst replacement in membrane reactors. Additionally, it could have secondary benefits of avoiding the use of immobilization chemistries that can be expensive and difficult to control and that sometimes can result in disappointing yields and/or activities of immobilized catalyst.

Many approaches exist for the immobilization of enzymes and homogeneous catalysts on solid supports. Several techniques including covalent bonding, crosslinking, entrapment, adsorption, and microencapsulation have been developed to render many enzymes water-insoluble. See Fig. 1A. Reviews of enzyme immobilization procedures have been published. Zaborsky, O.R., Immobilized Enzymes, CRC Press, Cleveland, Ohio (1973); Weetal, H. H., ed., Immobilized Enzymes, Antigens, Antibodies, and Peptides: Enzymology, Vol. 1, Marcel Dekker, N.Y. (1975); Gutcho, S. J., Immobilized Enzymes -- Preparation and Engineering Techniques, Noyes Data Corp., Park Ridge, N.H. (1974). Several industrial processes currently employ immobilized enzymes or immobilized whole cells. Mosbach, K., "Application of Immobilized Enzymes," pp. 717-858 in Immobilized Enzymes, K. Mosbach, ed., Methods in Enzymology XLIV. Academic Press, N.Y. (1976).

The possibility of immobilizing non-biological, ionic homogeneous catalysts as the counterions in ion exchange resins has been recognized for over thirty years. Helfferich, F., Ion Exchange, McGraw-Hill, N.Y. (1971). More recently, homogeneous catalyst complexes have been tied to polymeric and ceramic supports via bifunctional ligands which are simultaneously coordinated with the active metal center and anchored to the solid support. Pittman, C. U., and Evans, G. O. Chemtech, 3, 560 (1975); Michalska, Z. M., and Webster, D. E., Chemtech, 5, 117 (1975); Grubbs, R. H., Chemtech, 7, 512 (1977); Bailar, J. C., Jr., Cat. Rev. --Sci. Eng., 10(1), 17 (1974). Examples are shown in Figs. 1B and 1C.

Enzymes have been immobilized in membranes (as opposed to particles) in several different fashions. They have been covalently bound or crosslinked within porous membranes (Thomas, D., "Artificial enzyme membranes: transport, memory, and oscillatory phenomena," pp. 115-150 in Analysis and Control of Immobilized Enzyme Systems, D. Thomas and J. P. Kernevez, eds., American Elsevier, N.Y. (1976); Thomas, D., and Caplan, S. R., "Enzyme Membranes," pp. 351-398 in Membrane Separation Processes, P. Meares, ed., Elsevier, Amsterdam (1976); Fernandes, P.M., Constanides, A., Vieth, W. R., and Vendatasubramanian, K., Chemtech, 5, 438 (1975); Goldman, R., Kedem, O., and Katchalski, E., Biochem, 7, 4518 (1968)), attached to membrane surfaces (Emery, A., Sorenson, J., Kolarik, M., Swanson, S., and Lim, H., Biotechnol. Bioeng., 16, 1359 (1974)), entrapped in membrane gels (Blaedel, W. J., Kissel, T. R., and Bogulaski, R. C., Anal. Chem., 44, 2030 (1972); Blaedel, W. J., and Kissel, T. R., Anal. Chem., 47, 1602 (1975)), encapsulated by polymeric or liquid surfactant membrane microcapsules, (Chang, T. M. S., Artificial Cells, Charles C. Thomas, Springfield, IL (1972); Chang, T. M. S., and Kuntarian; N., pp. 193-7 in Enzyme Engineering 4, G. B. Brown, G. Manecke, and L. B. Wingard, eds., Plenum Press, NY (1978); May, S. W., and Landgraff, L. M., Biochem. Biophys. Res. Commun., 68,786 (1976); Mohan, R. R., and Li, N. N., Biotechnol. Bioeng., 16, 513 (1974).) and confined to reaction vessels by ultrafiltration membranes (Porter, M. C., "Applications of Membranes to Enzyme Isolation and Purification," pp. 115-144 in Enzyme Engineering 3, L. B. Wingard, ed., Interscience, N.Y. (1972); Closset, G. P., Cobb, J. T., and Shah, Y. T., Biotechnol. Bioeng., 16, 345 (1974); Madgavkar, A. M., Shah, Y. T., and Cobb, J. T., Biotechnol. Bioeng., 19, 1719 (1977)). The latter type of containment with membranes has been called "figurative immobilization" by Weetal (Messing, R. A., ed., Immobilized Enzymes for Industrial Reactors, Academic Press, NY (1975)), a term which also applies to the localization of an enzyme solution by hollow fibers (Rony, P. R., J. Am. Chem. Soc., 94, 8247 (1972); Davis, J. C., Biotechnol. Bioeng., 16, 1113 (1974); Lewis, W., and Middleman, S., AIChE J., 20, 1012 (1974); Waterland, L. R., Robertson, C. R., and Michaels, A. S., Chem. Eng. Commun., 2, 37 (1975)); U.S. Patent No. 4,266,026 to Breslau and 4,440,853 to Michaels (both all aqueous systems). Enzyme entrapment outside the fiber (i.e., within the "shell"), within the porous matrix, and in the fiber lumen have all been demonstrated in fully aqueous systems where reactants and products have been supplied and withdrawn, respectively, in aqueous process streams (see Figs. 2A, 2B, 2C).

Every conceivable membrane geometry -- planar films (Kay, T., Lilly, M. D., Sharp, A. K., and Wilson, R. J. H., Nature, 217, 641 (1968); Wilson, R. J. H., Kay, G., and Lilly, M. D., Biochem. J., 108, 845 (1968a); Wilson, R.J.H., Kay, G., and Lilly, M.D., Biochem. J., 109,137 (1968b)) and spiral-wrapped membranes (Vieth, W. R., Wang, S. S., Bernath, F. R., and Mogensen, A. O., "Enzyme Polymer Membrane Systems," pp. 175-202 in Recent Developments in Separation Science, Vol. 1, N. N. Li, ed., CRC Press, Cleveland, Ohio (1972); Broun, G., Thomas, D., Gellf, G., Domurado, D., Berjonneau, A. M., and Buillon, C., Biotechnol. Bioeng., 15, 359 (1973); Gautheron, D. C., and Coulet, P. R., pp. 123-7 in Enzyme Engineering 4, G. B. Broun, G. Manecke, and L. B. Wingard, eds., Plenum Press, NY (1978)), tubular membranes, (Madgavkar, A. M. Shah, Y. T., and Cobb, J. T., Biotechnol. Bioeng., 19, 1719 (1977); Tachauer, E., Cobb, J. T., and Shah, Y. T., Biotechnol. Bioeng., 16, 545 (1974)) and hollow fibers, asymetric hollow fibers having a single shell layer to retain a component, such as a catalyst, within the hollow fiber on one side of the process stream (as in U.S. Pat. No. 4,266,026 and U.S. Pat. No. 4,440,853) and microcapsules --and nearly all membrane types -- porous and nonporous, electrically charged and neutral --have been considered in connection with enzyme immobilization.

US-A-4 266 026 is considered as being the most relevant prior art.

## SUMMARY OF THE INVENTION

Briefly stated, the present invention operates by trapping a catalyst between two catalyst-impermeable boundaries that it cannot cross under normal membrane reactor operating conditions. These two barriers to catalyst transport are, generally speaking, (1) a "skin" or surface layer of said support membrane structure, which contains pores that are sufficiently small so as to prevent the transport and leakage of catalyst (which will often be either macromolecular or particulate in nature), and (2) a liquid-liquid phase boundary (e.g., between an aqueous solution entrapped in the pores of the membrane and an organic solvent residing just outside of it) that is located at the opposite surface of the catalyst-containing membrane structure. On one surface of the membrane structure, the size of the catalytic species prevents it from diffusing across the skin or surface layer of the asymmetric or composite membrane, while on the other surface the poor solubility of the catalyst in the immiscible liquid phase residing just outside of the membrane prevents loss of the catalytic species from that surface.

As an example, water-soluble enzymes used in two-phase and/or extractive membrane bioreactors could readily be contained in asymmetric, ultrafiltration-type membranes prepared from suitably hydrophilic polymers, where the membrane skin and the aqueous/organic phase boundary at opposite surfaces of the membrane would serve to confine the biocatalyst to the interior region of the water-wet porous membrane.

Alternatively, a two-layer composite structure consisting of a gel-type diffusion membrane (as used, for example, in dialysis) atop a microporous membrane support could also be employed for catalyst containment.

In cases where catalyst lifetime is short compared to that of the membrane support, the present invention makes possible the removal of deactivated catalyst and economical replacement thereof with active catalyst.

Few of the above-cited prior-art immobilization techniques bear much similarity to the present invention, either in structure or in function. Perhaps microencapsulation comes closest to the present invention, involving as it does a selective membrane barrier that prevents loss of catalyst from the interior; generally, selectivity is based on the size of the catalyst relative to the diameter of the pores in the microcapsule wall. However, microcapsules have but a single interface with the process stream (i.e., the microcapsule wall), and as a result the encapsulated catalyst is in contact with only a single process stream. In contrast, it is a purpose of the present invention to immobilize catalysts in membrane structures that permit close contact of the catalyst with multiple (and often immiscible) process streams.

## BRIEF DESCRIPTION OF THE FIGURES

The present invention may be more readily understood by reference to the following figures, wherein:

Figs. 1A, 1B, and 1C are schematic representations of commonly used catalyst immobilization techniques, with conventional methods for enzyme and for non-biological catalyst immobilization shown in Fig. 1A and Figs. 1B and 1C, respectively;

Figs. 2A, 2B and 2C are schematic representations of some conventional hollow fiber membrane/enzyme reactors as have been investigated for fully aqueous systems, with enzyme outside a fiber in a shell, enzyme in the porous matrix of a fiber, and enzyme in the lumen of a fiber shown in Figs. 2A, 2B and 2C, respectively;

Fig. 3 is a schematic representation of an illustrative embodiment of the invention in which a biocatalyst is contained within an inside-skinned, hydrophilic hollow fiber;

Fig. 4 is a schematic representation of an illustrative embodiment of the invention in which a non-biological homogeneous catalyst is contained within an outside-skinned, hydrophobic hollow fiber; and

Fig. 5 is a schematic representation of an illustrative embodiment of the invention in which a biocatalyst is contained within a composite, hydrophilic hollow fiber.

## DETAILED DESCRIPTION OF THE INVENTION

Fig. 3 shows a preferred embodiment of the present invention based on the use of a single asymmetric membrane with appropriate surface properties and wetting characteristics. Asymmetric membranes suitable for the practice of this invention are chosen from the group of anisotropic ultrafiltration (UF) and microfiltration (MF) membranes. These are characterized by a more-or-less thin "skin" layer, which in the case of UF membranes is on the order of 0.1-0.2 $\mu$m in thickness, supported atop a much thicker (100-200 $\mu$m) and highly porous substrate region. The skin of appropriate asymmetric UF- and MF-type membranes is characterized by sufficiently small pores (10's of $10^{-10}$m[Angstroms] to perhaps $100 \times 10^{-10}$m [Angstroms] in diameter) that macromolecular catalysts such as enzymes and colloidal or particulate catalysts are prevented from diffusing across to be lost to a process stream. Thus, the skin or surface region of the asymmetric membrane forms one catalyst-impermeable boundary.

The required characteristics of the "skin" layer will, of course, depend strongly on the size and other properties of the catalyst that is to be retained. In addition to the ultraporous (or even finely microporous) skin structures contemplated in the above paragraph, it may be advantageous in other situations to employ asymmetric membrane structures characterized by surface layers that resemble swollen gel-type membranes such as the type used in dialysis, or even to employ relatively "tight" membrane materials such as those used in thin-film-composite reverse osmosis membranes (generally for non-biologically catalyzed reactions).

The pores in the highly porous substrate region underlying the "skin" region of the membrane support can be and preferably are much larger (0.02 $\mu$m to several $\mu$m's in diameter) than those in the skin. The diameter of these substrate pores is chosen with two constraints in mind: (1) the pores must be large enough to accommodate the catalyst, which, in the case of whole cells, may be several microns in diameter and (2) the pores must be sufficiently small (a few microns at most) that capillary forces within them are significant. The latter consideration is important because the porous membrane substructure must be "wet" or impregnated by the "correct" liquid phase (e.g., usually the aqueous phase in the case of an enzyme-catalyzed conversion), and hence it is important that the intrusion pressure (the pressure at which the "incorrect" fluid can be forced into the pores of the substrate) not be exceeded during operation of the reactor. The intrusion pressure $\Delta P$ is inversely related to pore radius $r_{pore}$ by the Young-LaPlace equation:

$$\Delta P = (2\gamma/r_{pore})\cos\theta \qquad (1)$$

where $\gamma$ is the interfacial tension between the organic and aqueous phases and $\theta$ is the contact angle between the membrane material and the liquid phase contained therein. Typically, substrate pore sizes will be chosen such that the intrusion pressure is at least several kPa, to ensure stable operation of the membrane contained catalyst.

The second catalyst-impermeable boundary that defines the catalyst-containing region is defined by the liquid-liquid interface (typically, an aqueous/organic phase boundary) that is located at the surface of the membrane furthest removed from the "skin" layer. Capillarity acts to confine the desired liquid phase to the highly porous membrane matrix, and to exclude the other, immiscible liquid phase. Shown in Fig. 3 is the situation wherein the catalyst is Water-soluble or hydrophilic (i.e., preferentially wet by water), and the membrane material is chosen also to be hydrophilic. In this case, the aqueous/organic phase boundary will reside essentially at the outer, unskinned surface of the membrane, assuming that the pressure difference across the membrane is not in the direction so as to cause ultrafiltration of aqueous solution across the membrane or not so large as to cause intrusion of organic solvent into it. Under these circumstances, a water-soluble or hydrophilic catalyst will be confined to the aqueous interior of the membrane by virtue of its inability to partition into the organic solvent phase and subsequently be carried out of the reactor with it.

It is recognized that the relatively thick microporous substrate region that underlies the relatively thin skin layer of integrally skinned asymmetric membranes will often contain so-called macrovoids or "fingers" that are characterized by dimensions an order of magnitude or more larger than the diameter of the more prevalent micropores comprising the bulk of the substrate. Such macrovoid-containing asymmetric membranes are also within the scope of the present invention. For instance, for the particular case where the membrane is hydrophilic in nature, it is contemplated that the small micropores will be filled with aqueous catalyst-containing solution -- retained in the micropores by capillary action -- while the much larger macrovoids extending through the substrate will be filled with organic solvent. Intrusion of organic solvent into the macrovoids can be made to occur by applying a small amount of positive pressure to the organic phase sufficient to overcome the relatively small intrusion pressure associated with the larger diameter macrovoids (see Equation 1). In this manner, the area of aqueous/organic interface can be made larger than the superficial geometric area of the outer envelope of the membrane. For purposes of this disclosure, such area of the membrane where the aqueous/organic interface is located will be refered to as one of the surfaces of the membrane. Hydrophobic asymmetric membranes containing macrovoids can also be employed for the containment of organic-soluble catalyst in the microporous region of the substrate, the macrovoid being filled with aqueous solution in this case.

Several variations on this general theme can be identified. For example, the geometry of the asymmetric membrane is largely irrelevant to the present invention, whether the membrane be in the form of flat sheets, tubes, or hollow fibers (although the latter will usually be preferred from the points of view of manufacturability and cost). Moreover, both inside-skinned (as shown in Fig. 3) and outside-skinned hollow fibers, sheets or tubes may be employed, and the membrane material may be either hydrophilic (i.e., water-wet) or hydrophobic (i.e., preferentially wet by organic solvents). Considering just these two dimensions of skin location (i.e., inside- vs. outside-skinned) and surface properties (i.e., hydrophobic vs. hydrophilic), it is apparent that four different configurations can be identified, each of which will have its own set of advantages, disadvantages, and potential applications:

- o inside-skinned, hydrophilic,
- o inside-skinned, hydrophobic,
- o outside-skinned, hydrophilic, and
- o outside-skinned, hydrophobic.

For example, the outside-skinned and hydrophobic hollow-fiber of Fig. 4 may have utility in the conduct of phase-transfer catalyzed reactions, where the catalytic species is present predominantly in the organic phase. In this case, a macromolecular tail on the catalyst would assist in retaining it in the membrane matrix. The macromolecular tail may be comprised of, generally speaking, a macromolecule such as a polysaccharide, a protein, water soluble polymer (with hydrophilic membrane) or other polymer (i.e. polyethelene glycol). The macromolecular tails may be bonded or otherwise attached to the catalyst by standard methods known in the art such as covalent attachment using cyanogen bromide or glutaraldehyde.

The present invention can be further categorized according to the nature of the catalysts and reactions involved. For example, the present invention is useful both for localizing enzymes that are dissolved in the aqueous phase, as well as those that operate at aqueous/organic phase boundaries, such as certain of the lipases. Moreover, despite the focus of the above discussion, it is important to note that the utility of the invention is not limited to "bioconversions" such as those catalyzed by enzymes and viable or non-living whole cells. Various catalytic reactions of synthetic organic chemistry involve multiple phases (e.g., phase-transfer catalyzed reactions), and the present invention is equally useful in these cases. Finally, both soluble (typically, macromolecular) and particulate catalysts can be localized according to the method of the present invention.

The membrane structure of the present invention is operated in a diffusive mode, i.e., with diffusive transport of reactants into and products out of the catalytic region of the membrane; convective flow through the membrane is to be avoided. Preferably, reactants diffuse in on one side of the structure, and products diffuse out on the other, so that a separation and/or purification is accomplished simultaneously with the catalytic conversion.

The present invention is particularly useful in the conduct of catalyzed reactions in "multiphase" or "extractive" membrane reactors, where two process streams --one aqueous and one organic -- are located on opposite surfaces of the catalyst-containing membrane and serve the purpose of supplying reactant or removing product. For example, in cases where a reactant is soluble in organic solvents but not in water and where the reaction product is water soluble, the reactant may be fed to the reactor via a stream of organic solution directed past one surface of the membrane of the present invention, while the water-soluble product may be withdrawn from the opposite surface of the membrane via a second aqueous process stream. In other cases, a water-soluble reactant may be supplied via an aqueous stream directed past one surface of the membrane of the present invention while the product is made to partition and is thereby removed into a stream of organic solvent flowing past the other surface of the membrane.

The manner of loading the membrane with catalyst is illustrated here for the case of an enzymatic reaction conducted in the hydrophilic and inside-skinned hollow-fiber membrane of Fig. 3. Initially, aqueous enzyme solution is charged to the shell (or outer) side of the hollow-fiber module and passed though the fiber wall in an ultrafiltration process under a modest pressure difference (i.e., a pressure insufficient to cause disruption or loss of integrity of the skin under "back-flush" conditions). During this step, enzyme is accumulated in the porous substrate region of the fiber. Next, excess aqueous enzyme solution is displaced from the shell side of the fiber bundle by flushing it with an immiscible fluid such as air or the organic process solvent. If air or another gas is used in this step, the shell is filled with the organic solvent in a subsequent step. The module is then operated with the organic solvent on the shell side and an aqueous solution in the lumen of the fiber with a slight excess pressure on the shell side. This pressure difference is too small to cause intrusion of the organic phase into the substrate region of the fiber on the one hand and is in the wrong direction to cause ultrafiltration of aqueous solution on the other hand.

When the enzyme becomes deactivated and must be recharged, a positive pressure is applied to the aqueous solution on the interior or lumen side of the fibers, thereby causing ultrafiltration (i.e., convective flow) through the membrane and displacement both of organic solvent from the shell side of the module as well as deactivated enzyme from the fiber walls. In order to reload the membrane with catalyst, the steps of the preceding paragraph are repeated.

In another embodiment, a composite membrane structure is employed in place of the asymmetric, integrally skinned membranes contemplated above. As shown in Fig. 5, this composite consists of a thin, permselective surface layer of one material supported on a highly porous and much thicker nonselective substrate membrane, generally fashioned from a different material. Techniques for the fabrication of multilayer composite, laminated and coated membrane structures are well known in the art and are the subject of published review articles. Matson, S. L., Lopez, J. and J. A. Quinn, Chem. Eng. Sci., 38, 503 (1983); Lonsdale, H. K., J. Memb. Sci., 10, 81 (1982).

For example, composite membrane structures suitable for the localization of various biocatalysts might be manufactured based on the use of thin surface coatings of regenerated cellulose dialysis-type membrane supported on microporous membranes, particularly microporous hollow fibers. Alternatively, a suitably microporous layer might be deposited upon or within a regenerated cellulose hollow fiber. Hydrophilic polyacrylonitrile-based copolymer membranes also appear to be well suited to construction of such types of composite membrane structures.

The following examples are provided in order to further illustrate the invention disclosed herein.

ENZYME CONTAINMENT EXAMPLE

EXAMPLE 1

An enzyme solution was prepared by dissolving 50 grams of Candida lipase (Mol. Wt. 100,000; Sigma Chemical Co. Cat # L 1754) in 1.25 liters of water and then filtering this solution to remove the insoluble material. This inter-facially acting enzyme is known to hydrolyze a large number of organic esters, among them, phenoxyacetate methyl ester and amyl acetate.

The enzyme was loaded into a 1 $m^2$ custom-made solvent- resistant membrane module fabricated with anisotropic polyacrylonitrile (PAN) hollow fibers taken from a PAN-200 hemofilter (ASAHI Medical Co.). The morphology of this membrane is such that it can be described as an asymmetric hydrophilic inside-skinned hollow fiber characterized by 90% rejection of proteins with a molecular weight higher than 50,000. The enzyme solution was recirculated from the shell side to the lumen side and back to the solution reservoir in an ultrafiltration mode. Throughout the loading process the pressure difference between the shell and lumen compartments was kept to 55,12 kPa by adjusting the ultrafiltration rate (generally between 200 to 20 ml/min). The procedure was completed in one hour. The initial and final enzyme solution activities are shown below

| | Specific activity* µmole/min-ml | Total activity µmole/min |
|---|---|---|
| Initial solution | 7.84 | 9800 |
| Final solution | 0.05 | 63 |

* determined by measuring the rate of addition of 25 mM NaOH required to maintain the pH at 7.8 in a solution of 20 ml of 0.2 M NaCl + 0.5 ml of phenoxyacetate methyl ester (Aldrich Co.) + 2.5 ml of enzyme solution.

After loading the enzyme to the reactor, recirculation of 1140 ml of phenoxyacetate methyl ester on the shell side was started. The recirculation rate was 150 ml/min and the average pressure on the shell compartment was kept at 44,785 kPa by adjusting a throttling valve at the shell side exit. On the lumen side 2 liters of 0.1 M $NaHCO_3$ were recirculated at a rate of 300 ml/min. The pH of the aqueous reservoir was kept at 7.8 by addition of 50% NaOH. The reactor was run continuously for five days with daily replacement of the buffer and reaction- products solution in the aqueous-phase reservoir. Throughout the experiment, enzyme assays of the buffer reservoir showed no detectable enzymatic activity in the aqueous phase.

The reaction progress and rate were monitored by following the caustic consumption and observing the organic phase reservoir level. At the beginning of the experiment the rate of ester hydrolysis was 3000 µmoles/min and at the end it was 1500 µmoles/min. The phenoxyacetic acid product in the aqueous reservoirs was subsequently recovered by acidifying to a pH of 1.0 with concentrated HCl and filtering the precipitated solids. After drying, the solids were assayed titrimetrically and found to be 96.3 % acid. A sample of the dried solids was dissolved in a mixture of chloroform and water with a Subsequent drying/evaporation of the chloroform phase. The remaining solid from this purification step was titrimetrically assayed to be 99.5% pure with a melting point of 98-103 °C (melting point of phenoxy- acetic acid is 98-100 °C). The total amount of phenoxyacetic acid recovered was 0.953 kilogram.

EXAMPLE 2

Using the same membrane module described in Example 1 with the enzyme still in the membrane, the hydrolysis of amyl acetate was conducted. Recirculation of 400 ml of amyl acetate on the shell side was started and as before, the recirculation rate was 150 ml/min and the average pressure on the shell compartment was kept at 44,785 kPa by adjusting a throttling valve at the shell side exit. On the lumen side 1 liter of 0.05 M $NaHCO_3$ was recirculated at a rate of 300 ml/min. The pH of the aqueous reservoir was kept at 7.8 by addition of 5.57 H NaOH. The rate of amyl acetate hydrolysis was determined to be 250 µmoles per minute. Once the rate of amyl acetate hydrolysis was measured the reaction was stopped and the system rinsed with water both on the lumen and the shell side. The reactor was then backflushed for 15 hours with filtered (0.2 µm filter) tap water entering on the lumen side and exiting on the shell side at a flow rate of 50 ml/min. Two liters of 8 M urea were backflushed in the same manner as the tap water and then both, the shell and lumen compartments, were rinsed with 4 liters of distilled water.

The rate of amyl acetate hydrolysis in the reactor was measured in exactly the same manner described above with the exception that 25 mM NaOH was used. The reaction rate was 6.8 µmoles per minute which corresponds to 3% of the initial activity.

EXAMPLE 3

At the conclusion of the membrane regeneration procedure described in Example 2, the module was charged with 20 grams of Candida lipase (the same type and in the same concentration described in Example 1). Amyl acetate was used as a substrate and the reactor operated in exactly the same manner as described in Example 2. The rate of reaction was measured at 70 µmoles/min.

EXAMPLE 4

An enzyme solution was prepared by dissolving 10.8 ml of a pig liver esterase preparation (Mol. Wt. 150,000, 11 mg/ml, Sigma Chemical Co., Cat # E 3128) in 300 ml of 0.2 M phosphate buffer pH 8.0. This enzyme, falling under the category of an esterase, will hydrolyze ethyl butyrate dissolved in water, i.e., the reaction is a homogeneous one and does not require an organic/ aqueous interface to be present.

The enzyme was loaded into the same membrane module described in Example 1 by recirculating the enzyme solution from the shell side to the lumen side and back to the solution reservoir in an ultrafiltration mode. Throughout the loading process the pressure difference between the shell and lumen compartments was kept to 65,455 kPa by adjusting the ultrafiltration rate (generally between 200 to 20 ml/min). The procedure was completed in one hour. The initial and final enzyme solution activities are shown below

| | Specific activity* µmole/min-ml | Total activity µmole/min |
|---|---|---|
| Initial solution | 49.2 | 14800 |
| Final solution | 0.51 | 153 |

* determined by measuring the rate of addition of 20 mM NaOH required to maintain the pH at 8.0 in a solution of 20 ml of 0.1 M phosphate buffer pH 8.0 + 0.2 grams of ethyl butyrate (Aldrich Co.) + 1.0 ml of enzyme solution.

After loading the enzyme to the reactor, recirculation of 500 ml of ethyl butyrate on the shell side was started. The recirculation rate was 500 ml/min and the average pressure on the shell compartment was kept at 44,785 kPa by adjusting a throttling valve at the shell side exit. On the lumen side, 1 liter of 0.2 M phosphate buffer pH 8.0 was recirculated at a rate of 500 ml/min. The pH of the aqueous reservoir was kept at 8.0 by addition of 6.0 M NaOH. The rate of ethyl butyrate hydrolysis was determined to be 9600 umoles per minute. Once the rate of ethyl butyrate hydrolysis was measured, the reaction was stopped and the system rinsed with water both on the lumen and the shell side.

The enzyme was removed from the reactor through the following procedure:

- backflushed with 6 liters of distilled water entering on the lumen side and exiting on the shell side at a flow rate of 50 ml/min
- rinse on both the shell and lumen side with
  a) 4 liters of 1.0 M NaCl
  b) 500 ml of 12% $(NH_4)_2SO_4$.
  c) 500 ml of 8 M urea
  d) 2 liters of 1.0 M NaCl.

The rate of ethyl butyrate hydrolysis in the reactor was measured in exactly the same manner described above with the exception that 25 mM NaOH was used. The reaction rate was 30 µmoles per minute corresponding to 0.3% of the initial activity in the module.

EXAMPLE 5

An enzyme solution of alpha-Chymotrypsin (Mol. Wt. 23,000, Sigma Chemical Co. Cat # C 4129) was prepared by dissolving 0.5 grams of the enzyme in 1 liter of 0.1 M $K_2HPO_4$/1.0 M NaCl pH 7.8. This solution was recirculated on the shell side of a 1 $m^2$ ASAHI PAN-150 hemofilter (ASAHI Medical Co.) at a flowrate of 50 ml/min for 1 hour. Because there was no flow of enzyme solution from the shell to the lumen side, the enzyme was loaded into the membrane solely in a diffusive mode. After draining the shell side of the enzyme solution, recirculation of 1 liter of silicone oil (Petrarch Systems Inc.) was started on the shell compartment while maintaining a pressure of 62,01 kPa. A 0.2 mM solution of N-Benzoyl-L-Tyrosine Ethyl Ester (BTEE, Sigma Chemical Co.) in 0.1 M $K_2HPO_4$/l M NaCl pH 7.8 was then passed through the lumen side of the module at, a flow rate of 1 liter/min. The activity of the module was calculated by measuring the amount of BT acid that was present in the aqueous effluent from the reactor. The module activity was determined to be 80 µmoles/min. The membrane module was then drained of solvent and buffer and backflushed with 10 liters of 0.1 M phosphate buffer. The membrane module activity was measured in the same manner described above with the exception that the BTEE solution was pumped into the reactor at a rate of 53 ml/min. The activity of the membrane module was 4 µmole/min corresponding to 5% of the initial activity.

EXAMPLE 6

An enzyme solution was prepared by dissolving 100 mg of the same alpha-Chymotrypsin used in Example 4 in 500 ml of 0.1 M phosphate buffer pH 7.0. The enzyme was loaded on a 1 $m^2$ ASAHI PAN-150 hemofilter identical to the one used in Example 5 by recirculating the enzyme solution from the shell side to the lumen side and back to the solution reservoir in an ultra-filtration mode. The procedure was completed in 2.5 hours.

After loading the enzyme to the reactors recirculation of 1 liter of 10 mM BTEE in amyl acetate was started on the shell side. The recirculation rate was 10 ml/min and the average pressure on the shell compartment was kept at 44,785 kPa by adjusting a throttling valve at the shell side exit. On the lumen side, 200 ml of 2 mM phosphate buffer pH 7.0 was recirculated at a rate of 250 ml/min. The pH of the aqueous reservoir was maintained at 7.0 by addition of 1 M NaOH. The initial rate of BTEE hydrolysis was determined to be 45 µmoles/min.

EXAMPLE 7

In order to increase the amount of alpha-Chymotrypsin that is retained by the hollow fibers used in Examples 1-6, the molecular weight of the enzyme was increased by crosslinking with Bovine Serum Albumin (Sigma Chemical Co., Cat # A 4503) using glutaraldehyde and following conventional protocols for such chemistry. Gel permeation chromatography revealed that over 80% of the protein conjugate had a molecular weight in excess of 100,000. The final enzyme solution consisted of 1 liter of 0.1 M $K_2HPO_4$/l M NaCl pH 7.8 with a BTEE activity of 10 µmoles/min-ml. The enzyme was loaded on a 1 $m^2$ ASAHI PAN-150 hemofilter identical to the one used in Example 4 by ultrafiltering the solution once through from the shell side to the lumen side at a flowrate of 20 ml/min.

After loading the enzyme to the reactor, recirculation of 500 ml of 40 mM BTEE in n-octanol (Aldrich Co.) was started on the shell side. The recirculation rate was 500 ml/min and the average pressure on the shell compartment was kept at 44,785 kPa by adjustinq a throttling valve at the shell side exit. On the lumen side, 1 liter of 0.1 M $K_2HPO_4$/l M NaCl buffer pH 7.0 was recirculated at a rate of 500 ml/min. The pH of the aqueous reservoir was maintained at 7.0 by addition of 1 M NaOH. The initial rate of BTEE hydrolysis was determined to be 700 µmoles/min.

The invention described and claimed herein is not meant to be limited in scope by above experiments. Indeed, various modifications of the invention in addition to those shown and described herein will become apparent to those skilled in the art from the foregoing description. Such modifications are also intended to fall within the scope of the appended claims.

**Claims**

**1.** A method for confining a catalyst within a membrane between two fluid phases comprising:

(a) providing a membrane having

(i) a first surface characterized by a skin having pores that are large enough to allow permeation by reactants or products but small enough to substantially prevent catalyst leakage; and

(ii) a second surface characterized by pores that are large enough to allow permeation by reactants, products and catalyst;

(b) charging catalyst into the membrane by contacting the second surface of the membrane with catalyst in a first fluid that wets the membrane;

(c) contacting the first surface of the membrane with the first fluid; and

(d) replacing the first fluid used in charging the membrane at the second surface with a second fluid, which second fluid is substantially immiscible with the first fluid and in which the catalyst is not appreciably soluble ;

thereby confining the catalyst within the membrane between the two fluid phases.

**2.** A method for recharging an apparatus for confining a catalyst within a membrane between two fluid phases comprising:

(a) providing a catalyst-membrane wherein the catalyst has become deactivated having

(i) a first surface characterized by a skin having pores that are large enough to allow permeation by reactants or products but small enough to substantially prevent catalyst leakage, which first surface is in contact with a first fluid that wets the membrane; and

(ii) a second surface characterized by pores that are large enough to allow permeation by reactants, products and catalyst, which second surface is in contact with a second fluid that is substantially immiscible with the first fluid and in which the catalyst is not appreciably soluble;

(b) displacing the deactivated catalyst from the membrane by applying positive pressure to the first surface of the membrane;

(c) charging fresh catalyst into the membrane by contacting the second surface of the membrane with a solution of fresh catalyst in a fluid; and

(d) replacing the fluid used in charging the membrane at the second surface with the second fluid;

thereby recharging the apparatus.

**3.** A method of performing a catalytic reaction in which a reactant is supplied in one of first and second fluid streams and a product is removed in the other of said first and second fluid stream comprising the steps of:

providing a catalyst-containing membrane having:

(a) a first surface characterized by a skin having pores that are large enough to allow permeation by reactants or products but small enough to substantially prevent catalyst leakage, which first surface is in contact with a first fluid that wets the membrane; and

(b) a second surface characterized by pores that are large enough to allow permeation by reactants, products and catalyst, which second surface is in contact with a second fluid that is substantially immiscible with the first fluid and in which the catalyst is not appreciably soluble;

whereby the catalyst is confined between the two fluid phases;

introducing said reactant in one of said first and second fluids; and

removing said product in the other of said first and second fluids.

**4.** The method of claim 3 further comprising the steps of charging catalyst into the membrane by contacting the second surface of the membrane with a solution of said catalyst in a fluid, and replacing the fluid used in charging the membrane at the second surface with said second fluid.

**5.** A method according to any of the claims 1-4 wherein said membrane is an asymmetric membrane.

**6.** A method according to any of the claims 1-4 wherein said membrane is a composite membrane.

**7.** A method according to any of the claims 1-4 wherein said membrane is in the form of a flat sheet.

**8.** The method of claim 7 wherein said membrane is of a hydrophilic material and the first fluid is an aqueous solution.

**9.** The method of claim 7 wherein said membrane is of a hydrophobic material and the first fluid is a substantially water-immiscible organic solvent.

**10.** A method according to any of the claims 1-4 wherein said membrane is in the form of a tube.

**11.** The method of claim 10 wherein said membrane is of a hydrophilic material, the first surface is at the interior of the tube, and the first fluid is an aqueous solution.

**12.** The method of claim 10 wherein said membrane is of a hydrophilic material, the second surface is at the interior of the tube, and the second fluid is a substantially water-immiscible organic solvent.

13. The method of claim 10 wherein said membrane is of a hydrophobic material, the first surface is at the interior of the tube and the first fluid is a substantially water-immiscible organic solvent.

14. The method of claim 10 wherein said membrane is of a hydrophobic material, the second surface is at the interior of the tube, and the second fluid is an aqueous solution.

15. A method according to any of the claims 1-4 wherein said membrane is in the form of a hollow fiber.

16. The method of claim 15 wherein said membrane is of a hydrophilic material, the first surface is at the lumen, and the first fluid is an aqueous solution.

17. The method of claim 15 wherein said membrane is of a hydrophilic material, the second surface is at the lumen, and the second fluid is a substantially water-immiscible organic solvent.

18. The method of claim 15 wherein said membrane is of a hydrophobic material, the first surface is at the lumen, and the first fluid is a substantially water-immiscible organic solvent.

19. The method of claim 15 wherein said membrane is of a hydrophobic material, the second surface is at the lumen, and the second fluid is an aqueous solution.

20. A method according to any of the claims 1-4 wherein the catalyst is a homogenous non-biological catalyst.

21. A method according to any of the claims 1-4 wherein the catalyst is an enzyme.

22. A method according to any of the claims 1-4 wherein the catalyst is attached to a macromolecule.

23. A method according to any of the claims 1-4 wherein the catalyst is attached to a polysaccharide.

24. A method according to any of the claims 1-4, wherein the catalyst is attached to a protein.

25. A method according to any of the claims 1-4, wherein the catalyst is attached to a polymer.

26. A method according to any of the claims 1-4, wherein the catalyst is attached to a water-soluble polymer.

27. A method of claims 22-26 wherein the catalyst is attached covalently.

28. A method according to any of the claims 1-4 wherein the catalyst is a whole cell.

29. The method according to claims 3 and 4 wherein a reactant is supplied in the first fluid and a product is removed in the second fluid.

30. The method according to claims 3 and 4 wherein a reactant is supplied in the second fluid and a product is removed in the first fluid.

## Patentansprüche

1. Ein Verfahren, bei dem ein Katalysator zwischen zwei Flüssigkeitsphasen in eine Membrane eingeschlossen wird, und das besteht aus:
   (a) einer Membrane mit
   (i) einer ersten Fläche, die durch eine Hautschicht mit Poren gekennzeichnet wird, die groß genug sind, um Reaktionsbestandteile oder -produkte durchdringen zu lassen, die jedoch auch klein genug sind, um ein Herauslecken von Katalysator nahezu vollständig zu verhindern, und
   (ii) einer zweiten Fläche, die durch eine Hautschicht mit Poren gekennzeichnet wird, die groß genug sind, um das Durchdringen von Reaktionsbestandteilen, Reaktionsprodukten und Katalysator zu ermöglichen;

(b) dem Laden von Katalysator in die Membrane, und zwar dadurch, daß die zweite Fläche der Membrane in einer ersten die Membrane benetzenden Flüssigkeit mit Katalysator in Kontakt gebracht wird;
(c) dem in Kontakt bringen der ersten Fläche der Membrane mit der ersten Flüssigkeit; und
(d) dem Ersetzen der ersten Flüssigkeit, die zum Füllen der Membrane an der zweiten Fläche benutzt wurde, durch ein zweite Flüssigkeit, wobei diese zweite Flüssigkeit nahezu nicht mit der ersten Flüssigkeit vermischbar ist, und in dem der Katalysator nicht wahrnehmbar löslich ist;
wobei der Katalysator zwischen den zwei Flüssigkeitsphasen in der Membrane eingeschlossen bleibt.

**2.** Ein Verfahren, bei dem eine Vorrichtung zum Einschließen eines Katalysators zwischen zwei Flüssigkeitsphasen in eine Membrane erneut gefüllt wird, und das besteht aus:
(a) einer Katalysatormembrane, in dem der Katalysator inaktiviert wurde, mit
(i) einer ersten Fläche, die durch eine Hautschicht mit Poren gekennzeichnet wird, die groß genug sind, um Reaktionsbestandteile oder -produkte durchdringen zu lassen, die jedoch auch klein genug sind, um ein Herauslecken von Katalysator nahezu vollständig zu verhindern, wobei diese erste Fläche mit einer ersten, die Membrane benetzenden Flüssigkeit in Kontakt steht, und
(ii) einer zweiten Fläche, die durch eine Hautschicht mit Poren gekennzeichnet wird, die groß genug sind, um das Durchdringen von Reaktionsbestandteilen, Reaktionsprodukten und Katalysator zu ermöglichen, wobei diese zweite Fläche mit einer zweiten Flüssigkeit in Kontakt steht, die nahezu nicht mit der ersten Flüssigkeit vermischbar ist, und in dem der Katalysator nicht wahrnehmbar löslich ist;
(b) dem Abführen des inaktivierten Katalysators aus der Membrane durch das Ausüben eines positiven Drucks auf die erste Fläche der Membrane;
(c) dem Laden von neuem Katalysator in die Membrane, und zwar dadurch, daß die zweite Fläche der Membrane mit einer Lösung von neuem Katalysator in einer Flüssigkeit in Kontakt gebracht wird; und
(d) dem Ersetzen der ersten Flüssigkeit, die zum Füllen der Membrane an der zweiten Fläche benutzt wurde, durch ein zweite Flüssigkeit;
wobei die Vorrichtung somit erneut gefüllt wird.

**3.** Ein Verfahren zum Durchführen einer katalytischen Reaktion, bei der ein Reaktionsbestandteil in einer ersten oder in einer zweiten Flüssigkeitsströmung zugeführt wird, und bei dem ein Reaktionsprodukt in der jeweils anderen Flüssigkeitsströmung abgeführt wird, wobei das Verfahren aus den folgenden Schritten besteht:
der Schaffung einer Katalysator enthaltenden Membrane mit:
(a) einer ersten Fläche, die durch eine Hautschicht mit Poren gekennzeichnet wird, die groß genug sind, um Reaktionsbestandteile oder -produkte durchdringen zu lassen, die jedoch auch klein genug sind, um ein Herauslecken von Katalysator nahezu vollständig zu verhindern, wobei diese erste Fläche mit einer ersten, die Membrane benetzenden Flüssigkeit in Kontakt steht, und
(b) einer zweiten Fläche, die durch eine Hautschicht mit Poren gekennzeichnet wird, die groß genug sind, um das Durchdringen von Reaktionsbestandteilen, Reaktionsprodukten und Katalysator zu ermöglichen, wobei diese zweite Fläche mit einer zweiten Flüssigkeit in Kontakt steht, die nahezu nicht mit der ersten Flüssigkeit vermischbar ist, und in dem der Katalysator nicht wahrnehmbar löslich ist;
wobei der Katalysator zwischen den zwei Flüssigkeitsphasen eingeschlossen ist;
und bei dem Reaktionsbestandteil in die ersten oder in die zweiten Flüssigkeitsströmungen eingebracht wird, und
das Reaktionsprodukt in die jeweils anderen Flüssigkeitsströmungen abgeführt wird.

**4.** Ein Verfahren nach Anspruch 3, bei dem das Verfahren ansonsten noch die Schritte enthält, in denen Katalysator in die Membrane geladen wird, indem die zweite Fläche der Membrane mit einer Lösung des Katalysators in einer Flüssigkeit in Kontakt gebracht wird, und bei dem die beim Füllen der Membrane an der zweiten Fläche benutzte Flüssigkeit durch die zweite Flüssigkeit ersetzt wird.

**5.** Ein Verfahren nach einem der Ansprüche 1 - 4, bei dem die Membrane eine asymmetrische Membrane ist.

6. Ein Verfahren nach einem der Ansprüche 1 - 4, bei dem die Membrane eine zusammengesetzte Membrane ist.

7. Ein Verfahren nach einem der Ansprüche 1 - 4, bei dem die Membrane in Form einer flachen Platte ausgeführt ist.

8. Ein Verfahren nach Anspruch 7, bei dem die Membrane aus hydrophilem Material besteht, und bei dem die erste Flüssigkeit eine Lösung in Wasser ist.

9. Ein Verfahren nach Anspruch 7, bei dem die Membrane aus hydrophobem Material besteht, und bei dem die erste Flüssigkeit ein nahezu nicht mit Wasser vermischbares organisches Lösungsmittel ist.

10. Ein Verfahren nach einem der Ansprüche 1 - 4, bei dem die Membrane in Form eines Rohres ausgeführt ist.

11. Ein Verfahren nach Anspruch 10, bei dem die Membrane aus hydrophilem Material besteht, die erste Fläche sich an der Innenseite des Rohres befindet und die erste Flüssigkeit eine Lösung in Wasser ist.

12. Ein Verfahren nach Anspruch 10, bei dem die Membrane aus hydrophilem Material besteht, die zweite Fläche sich an der Innenseite des Rohres befindet und die zweite Flüssigkeit eine nahezu nicht mit Wasser vermischbares organisches Lösungsmittel ist.

13. Ein Verfahren nach Anspruch 10, bei dem die Membrane aus hydrophobem Material besteht, die erste Fläche sich an der Innenseite des Rohres befindet und die erste Flüssigkeit eine nahezu nicht mit Wasser vermischbares organisches Lösungsmittel ist.

14. Ein Verfahren nach Anspruch 10, bei dem die Membrane aus hydrophobem Material besteht, die zweite Fläche sich an der Innenseite des Rohres befindet und die zweite Flüssigkeit eine Lösung in Wasser ist.

15. Ein Verfahren nach einem der Ansprüche 1 - 4, bei dem die Membrane in Form einer hohlen Faser ausgeführt ist.

16. Ein Verfahren nach Anspruch 15, bei dem die Membrane aus hydrophilem Material besteht, die erste Fläche sich am Hohlraum befindet und die erste Flüssigkeit eine Lösung in Wasser ist.

17. Ein Verfahren nach Anspruch 15, bei dem die Membrane aus hydrophilem Material besteht, die zweite Fläche sich am Hohlraum befindet und die zweite Flüssigkeit ein nahezu nicht mit Wasser vermischbares organisches Lösungsmittel ist.

18. Ein Verfahren nach Anspruch 15, bei dem die Membrane aus hydrophobem Material besteht, die erste Fläche sich am Hohlraum befindet und die erste Flüssigkeit ein nahezu nicht mit Wasser vermischbares organisches Lösungsmittel ist.

19. Ein Verfahren nach Anspruch 15, bei dem die Membrane aus hydrophobem Material besteht, die zweite Fläche sich am Hohlraum befindet und die zweite Flüssigkeit eine Lösung in Wasser ist.

20. Ein Verfahren nach einem der Ansprüche 1 - 4, bei dem der Katalysator ein homogener, nicht biologischer Katalysator ist.

21. Ein Verfahren nach einem der Ansprüche 1 - 4, bei dem der Katalysator ein Enzym ist.

22. Ein Verfahren nach einem der Ansprüche 1 - 4, bei dem der Katalysator an ein Makromolekül gebunden wird.

23. Ein Verfahren nach einem der Ansprüche 1 - 4, bei dem der Katalysator an ein Polysaccharid gebunden wird.

**24.** Ein Verfahren nach einem der Ansprüche 1 - 4, bei dem der Katalysator an ein Protein gebunden wird.

**25.** Ein Verfahren nach einem der Ansprüche 1 - 4, bei dem der Katalysator an ein Polymer gebunden wird.

**26.** Ein Verfahren nach einem der Ansprüche 1 - 4, bei dem der Katalysator an ein in Wasser lösliches Polymer gebunden wird.

**27.** Ein Verfahren nach den Ansprüchen 22 - 26, bei dem eine kovalente Bindung des Katalysators stattfindet.

**28.** Ein Verfahren nach einem der Ansprüche 1 - 4, bei dem der Katalysator eine ganze Zelle ist.

**29.** Ein Verfahren nach den Ansprüchen 3 und 4, bei dem ein Reaktionsbestandteil in der ersten Flüssigkeit zugeführt und ein Reaktionsprodukt in der zweiten Flüssigkeit abgeführt wird.

**30.** Ein Verfahren nach den Ansprüchen 3 und 4, bei dem ein Reaktionsbestandteil in der zweiten Flüssigkeit zugeführt und ein Reaktionsprodukt in der ersten Flüssigkeit abgeführt wird.

## Revendications

**1.** Une méthode pour le confinement d'un catalyseur dans une membrane entre deux phases fluides comprenant :

(a) une membrane présentant

(i) une première surface caractérisée par une peau comportant des pores qui sont assez grands pour permettre la perméation par des réactants ou des produits, mais assez petits pour empêcher substantiellement la fuite de catalyseur et

(ii) une seconde surface caractérisée par des pores qui sont assez grands pour permettre la perméation par des réactants, produits et catalyseur ;

(b) le chargement d'un catalyseur dans la membrane en mettant en contact la seconde surface de la membrane avec un catalyseur dans un premier fluide qui humidifie la membrane ;

(c) mise en contact de la première surface de la membrane avec le premier fluide, et

(d) remplacement du premier fluide utilisé pour charger la membrane au niveau de la seconde surface par un second fluide, lequel est substantiellement non miscible avec le premier fluide et dans lequel le catalyseur n'est pas soluble de façon appréciable ;

confinant ainsi le catalyseur dans la membrane entre les deux phases fluides.

**2.** Une méthode de recharge d'un dispositif pour le confinement d'un catalyseur dans une membrane entre les deux phases fluides comprenant :

(a) la présence d'une membrane catalysatrice dans laquelle le catalyseur se trouve désactivé et présentant

(i) une première surface caractérisée par une peau comportant des pores qui sont assez grands pour permettre la perméation par des réactants ou des produits, mais assez petits pour empêcher substantiellement la fuite du catalyseur, laquelle première surface est en contact avec un premier fluide qui humidifie la membrane, et

(ii) une seconde surface caractérisée par des pores qui sont assez grands pour permettre la perméation par des réactants, produits et catalyseur, laquelle seconde surface est en contact avec le second fluide qui est substantiellement non miscible avec le premier fluide et dans lequel le catalyseur n'est pas soluble de façon appréciable ;

(b) retrait du catalyseur désactivé de la membrane en appliquant une pression positive sur la première surface de la membrane ;

(c) chargement d'un nouveau catalyseur dans la membrane en mettant en contact la seconde surface de la membrane avec une solution de nouveau catalyseur dans un fluide, et

(d) remplacement du fluide utilisé dans le chargement de la membrane en sa seconde surface par le second fluide ;

rechargeant ainsi le dispositif.

3. Une méthode d'exécution d'une réaction catalytique dans laquelle un réactant est introduit dans l'un des premier et second courants de fluides et un produit est retiré dans l'autre desdits premier et second courants de fluide, comprenant les stades de fourniture d'une membrane contenant un catalyseur ayant:
   (i) une première surface caractérisée par une peau comportant des pores qui sont assez grands pour permettre la perméation par des réactants ou des produits, mais assez petits pour empêcher substantiellement la fuite de catalyseur, laquelle première surface est en contact avec un premier fluide qui humidifie la membrane, et
   (ii) une seconde surface caractérisée par des pores qui sont assez grands pour permettre la perméation par des réactants, produits et catalyseur, laquelle seconde surface est en contact avec un second fluide qui est substantiellement non miscible avec le premier fluide et dans lequel le catalyseur n'est pas soluble de manière appréciable ;
   grâce à laquelle le catalyseur est confiné entre les deux phases fluides ;
   l'introduction dudit réactant dans l'un desdits premier ou second fluides ; et
   le retrait dudit produit dans l'autre desdits premier et second fluides.

4. La méthode de la revendication 3 comporte en outre les stades de chargement du catalyseur dans la membrane par mise en contact de la seconde surface de la membrane avec une solution dudit catalyseur dans un fluide et le remplacement du fluide utilisé en chargeant la membrane en sa seconde surface à l'aide d'un second fluide.

5. Une méthode selon l'une quelconque des revendications 1 à 4 où ladite membrane est une membrane asymétrique.

6. Une méthode selon l'une quelconque des revendications 1 à 4 où ladite membrane est une membrane composite.

7. Une méthode selon l'une quelconque des revendications 1 à 4 où ladite membrane se présente sous la forme d'un feuillet plat.

8. La méthode de la revendications 7 où ladite membrane est faite d'un matériau hydrophile et le premier fluide est une solution aqueuse.

9. La méthode de la revendications 7 où ladite membrane est faite d'un matériau hydrophobe et le premier fluide est un solvant organique substantiellement non miscible à l'eau.

10. Une méthode selon l'une quelconque des revendications 1 à 4 où ladite membrane se présente sous la forme d'un tube.

11. La méthode de la revendications 10 où ladite membrane est faite d'un matériau hydrophile, la première surface se trouve à l'intérieur du tube et le premier fluide est une solution aqueuse.

12. La méthode de la revendication 10 où ladite membrane est faite d'un matériau hydrophile, la seconde surface est à l'intérieur du tube et le second fluide est un solvant organique substantiellement non miscible à l'eau.

13. La méthode de la revendication 10 où ladite membrane est faite d'un matériau hydrophobe, la première surface est à l'intérieur du tube et le premier fluide est un un solvant organique substantiellement non miscible à l'eau.

14. La méthode de la revendications 10 où ladite membrane est faite d'un matériau hydrophobe, la seconde surface est à l'intérieur du tube et le second fluide est une solution aqueuse.

15. Une méthode selon l'une quelconque des revendications 1 à 4 où ladite membrane se présente sous la forme d'une fibre creuse.

16. La méthode de la revendications 15 où ladite membrane est faite d'un matériau hydrophile, la première surface se trouve à la lumière et le premier fluide est une solution aqueuse.

**17.** La méthode de la revendication 15 où ladite membrane est faite d'un matériau hydrophile, la seconde surface est à la lumière et le second fluide est un solvant organique substantiellement non miscible à l'eau.

**18.** La méthode de la revendication 15 où ladite membrane est faite d'un matériau hydrophobe, la première surface est à la lumière et le premier fluide est un solvant organique substantiellement non miscible à l'eau.

**19.** La méthode de la revendications 15 où ladite membrane est faite d'un matériau hydrophobe, la seconde surface est à la lumière et le second fluide est une solution aqueuse.

**20.** Une méthode selon l'une quelconque des revendications 1 à 4 où ledit catalyseur est un catalyseur non biologique homogène.

**21.** Une méthode selon l'une quelconque des revendications 1 à 4 où ledit catalyseur est une enzyme.

**22.** Une méthode selon l'une quelconque des revendications 1 à 4 où ledit catalyseur est lié à une macromolécule.

**23.** Une méthode selon l'une quelconque des revendications 1 à 4 où ledit catalyseur est lié à un polysaccharide.

**24.** Une méthode selon l'une quelconque des revendications 1 à 4 où ledit catalyseur est lié à une protéine.

**25.** Une méthode selon l'une quelconque des revendications 1 à 4 où ledit catalyseur est lié à un polymère.

**26.** Une méthode selon l'une quelconque des revendications 1 à 4 où ledit catalyseur est lié à un polymère soluble dans l'eau.

**27.** Une méthode selon les revendications 22 à 26 où le catalyseur est lié de manière covalente.

**28.** Une méthode selon l'une quelconque des revendications 1 à 4 où ledit catalyseur est une cellule entière.

**29.** La méthode selon les revendications 3 et 4 où un réactant est introduit dans le premier fluide et un produit est retiré dans le second fluide.

**30.** La méthode selon les revendications 3 et 4 où un réactant est introduit dans le second fluide et un produit est retiré dans le premier fluide.

FIG. 1A

Prior Art

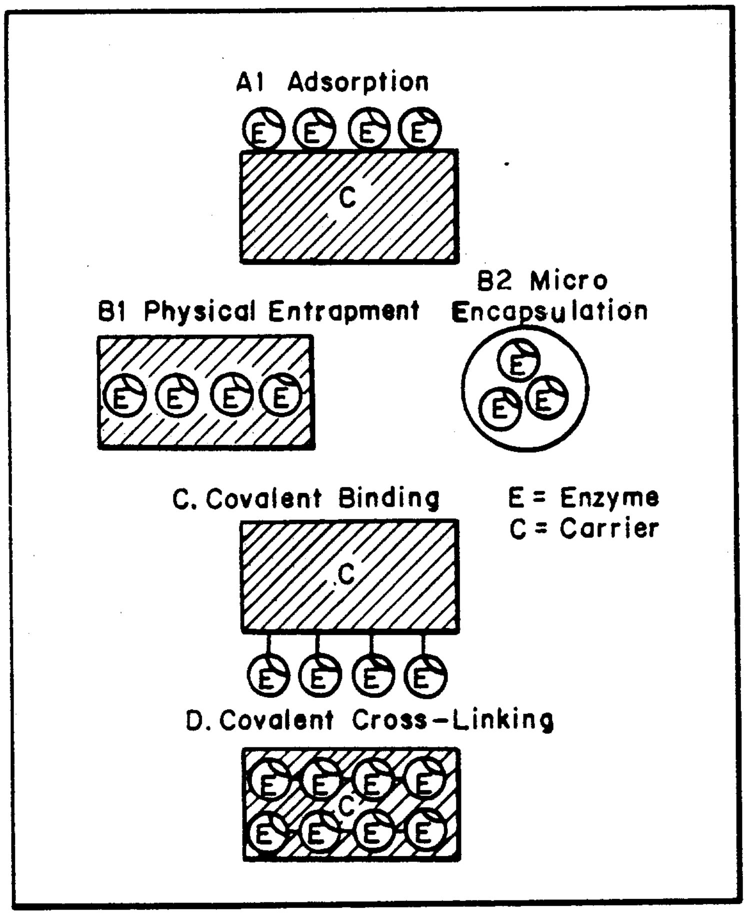


FIG. 1B

Prior Art

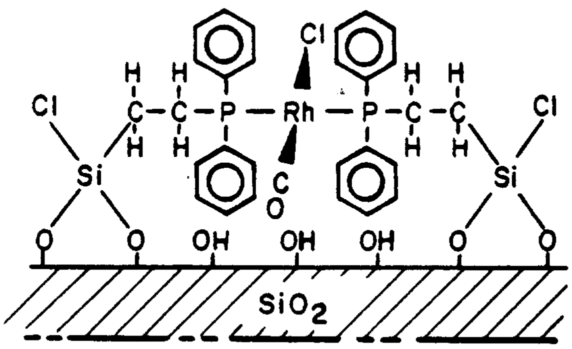

FIG. 1C

Prior Art

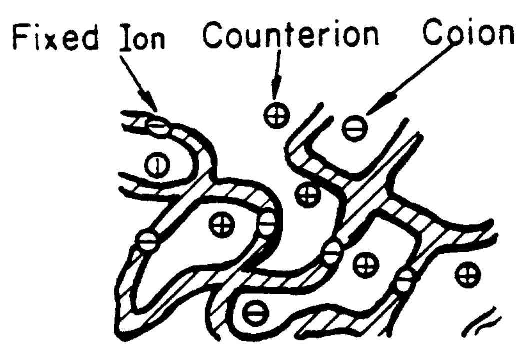

FIG. 2A
Prior Art

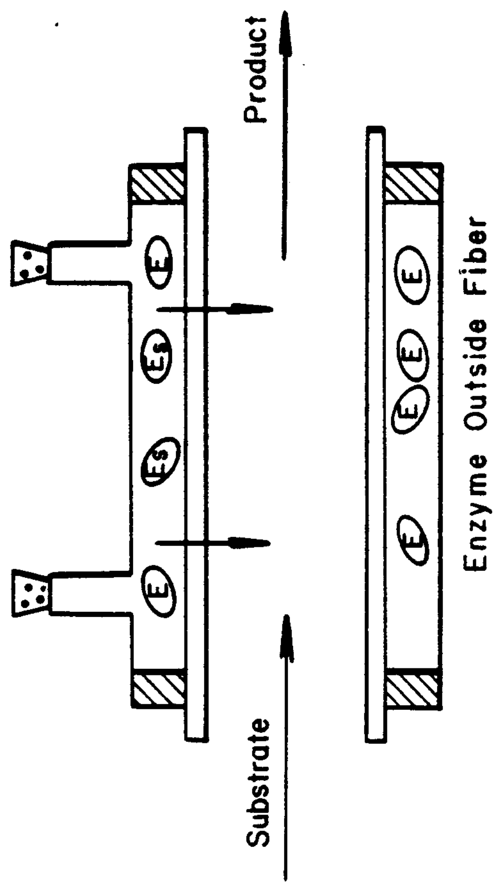


FIG. 2C
Prior Art

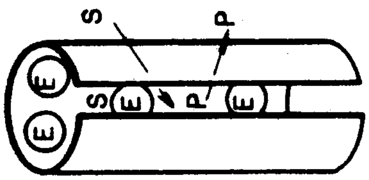


FIG. 2B
Prior Art

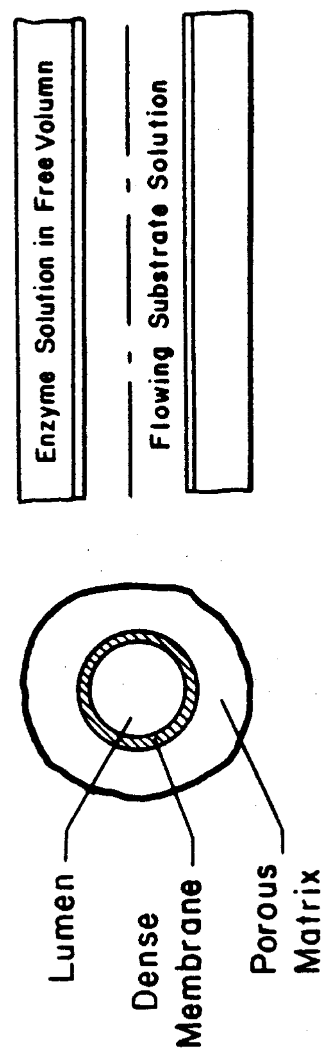

FIG. 3

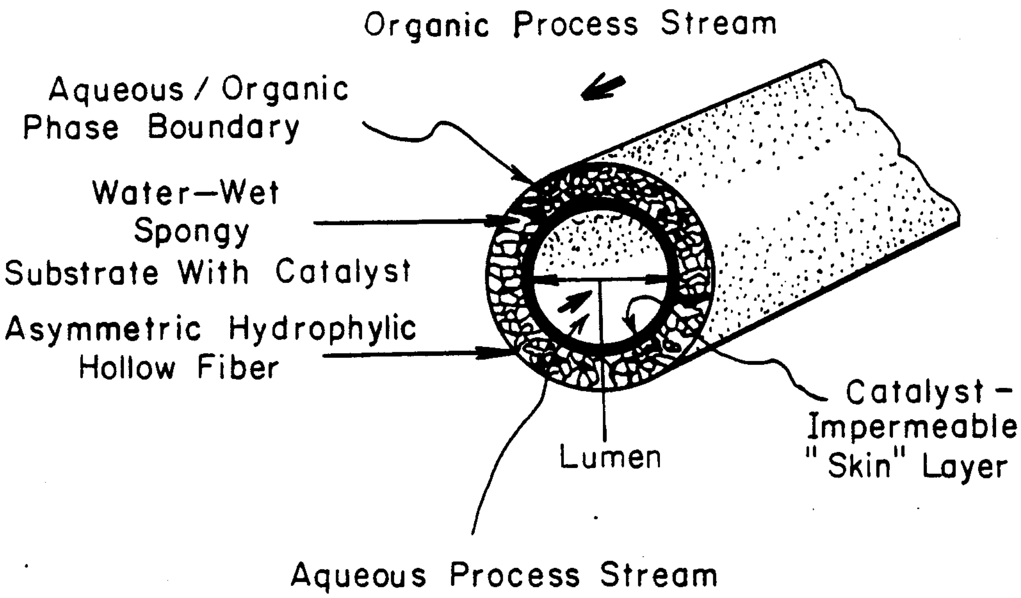
Organic Process Stream
Aqueous / Organic
Phase Boundary
Water—Wet
Spongy
Substrate With Catalyst
Asymmetric Hydrophylic
Hollow Fiber
Catalyst -
Impermeable
"Skin" Layer
Lumen
Aqueous Process Stream

FIG. 4

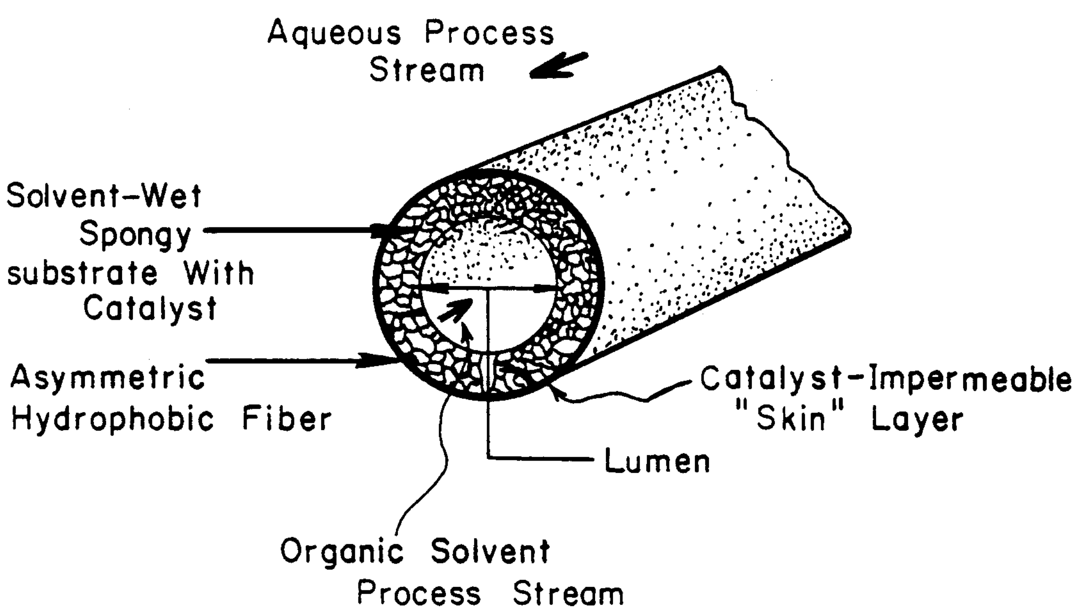
Aqueous Process
Stream
Solvent-Wet
Spongy
substrate With
Catalyst
Asymmetric
Hydrophobic Fiber
Catalyst-Impermeable
"Skin" Layer
Lumen
Organic Solvent
Process Stream

FIG. 5

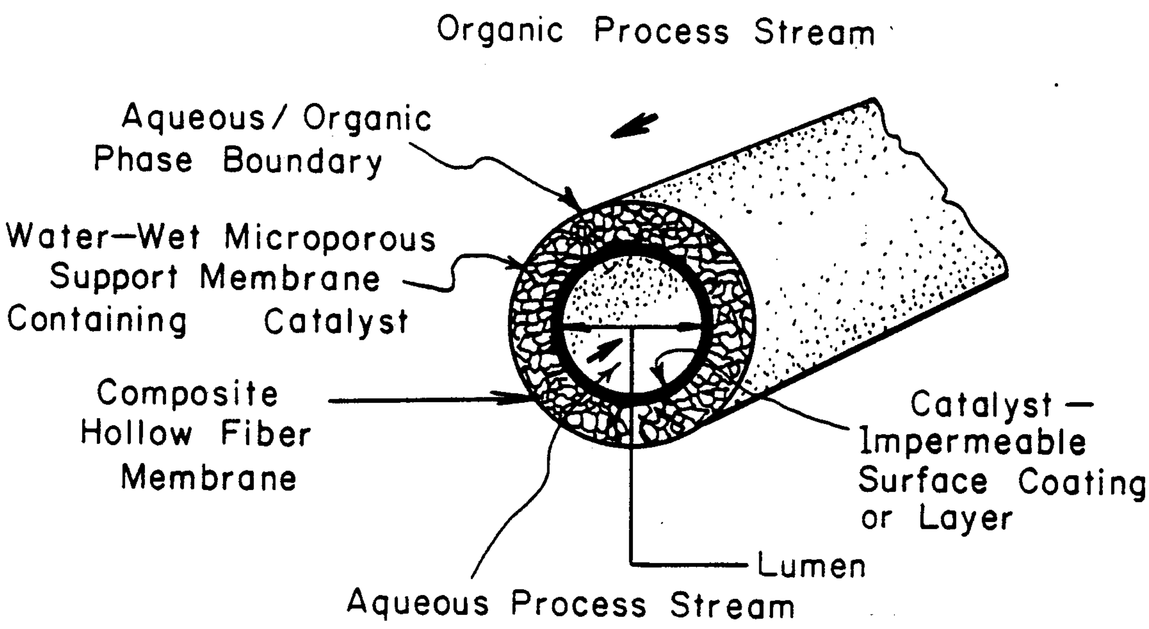
Organic Process Stream
Aqueous/ Organic Phase Boundary
Water–Wet Microporous Support Membrane Containing Catalyst
Composite Hollow Fiber Membrane
Catalyst — Impermeable Surface Coating or Layer
Lumen
Aqueous Process Stream